# EUROPEAN PATENT SPECIFICATION

(11) **EP 1 390 899 B1**
(45) Date of publication and mention of the grant of the patent: **29.03.2006**
(21) Application number: 02764111.7
(22) Date of filing: 22.04.2002
(51) Int. Cl.: G06F 19/00

(54) **METHOD FOR SAFE TRANSFER OF PATIENT DATA ON A DATA CARRIER**
VERFAHREN ZUM SICHEREN TRANSFER VON PATIENTENDATEN AUF EINEM DATENTRÄGER
PROCEDE DE TRANSFERT SECURISE DE DONNEES SUR LES PATIENTS DANS UNE PORTEUSE DE DONNEES

(30) Priority: 25.04.2001 NO 20012023
(43) Date of publication of application: 25.02.2004
(73) Proprietor: World Medical Center (Holding) SA, 1297 Founex (CH)
(72) Inventor: VEIDUNG, Arne, N-5824 Bergen (NO)
(74) Representative: Plougmann & Vingtoft A/S
(86) International application number: PCT/NO2002/000149
(87) International publication number: WO 2002/086791

(56) References cited:
- EP-A1- 0 423 893
- WO-A1-96/15503
- WO-A2-00/65522
- DE-A1- 19 840 005
- US-A- 5 924 074
- US-A- 5 930 759
- US-A- 6 031 910

## Description

The present invention relates to a method for safe transfer of patient data on a data carrier, where patient data is coded and transferred by means of a network to a central server comprising a database, said patient data being stored in a storage unit in the server. Coded data is transformed and printed on a data carrier which is kept by the patient, and coded data is being read from the data carrier by means of a reading unit and transformed to a legible form by a decoder.

From EP-A1 423893 it is known a method for storing and monitoring of patient related information, in health centre, wherein each patient is provided with at patient connected electronic data carrier, which can be read out and reprogrammed, Patent related data are stored both locally in the patient connected electronic data carrier and centrally in a central computer. Locally stored data are compared in predetermined situations with the centrally stored data and the two types of data are, if necessary, matched.

A similar system is known from DE A1 19840005, which discloses a communication system with an input device and an output device, and a memory-storing unit, holding patient mailboxes for input and output. The input device is connected through an interface to terminals for bar-coded form cards, patient chip cards and through another interface to the memory storage unit by means of a communication terminal. The system is to be used in hospitals and medical administrative systems for identifying details about patients .

The present invention, as claimed, is an international concept that puts the safety of the patient in focus when the patient is undergoing any medical treatment. With the system according to the present invention a better diagnosis and basis for treatment can be provided, and thereby the correct medication be given wherever the patient may be. It is often a problem in acute cases that the attending physician does not know anything about the patient's previous illnesses, uses of medication or allergies/reactions to medication. This is particularly relevant when being taken ill abroad.

Such vital information can be decisive in avoiding the giving of the wrong treatment and the wrong medication. This is a problem, which is steadily increasing and unfortunately can lead to loss of life for patients. In the US, it is defined as a society problem.

An object of the present invention is to make patient data available to any physician who is treating the patient. In addition, the system shall also ensure complete protection of the patient's privacy. The founder of this system visualises that a web-based service centre is established in every country such that a network of local physicians, ambulance systems, emergency wards and hospitals is set up. These are authorised and are equipped with the programs and equipment that are necessary for interactive web-communication with a central server.

An important part of the system is that the patient's regular physician keeps the medical journal and that essential elements of this are loaded onto a structured medicine card. The card will contain the patient's diagnoses, illnesses/injuries, use of medication and allergies or reactions to medication. After registration, the physician provides the patient with a copy of the information on the card, which the patient is given to have control over his/her own data. Thereafter, the physician sends the information on the card by means of a central server where the data is stored in a database. Before sending, the data is automatically encrypted so that the patient's privacy is ensured.

The registered patient will receive a new copy of data, which is loaded into the database as a control of the copy, which is received by the physician. Furthermore, the patient receives a two-dimensional code, which contains the same information. According to the present invention, this code is very important in that the patient will have the necessary data with him at all times. The code can be fixed to a self-adhesive means, which, for example, can be stuck to the back of a watch and/or on a card to be kept together with other cards.

The advantage of the system is that it can function as quality assurance for physicians or nurses in daily treatments in institutions, in home-help situations or during ordinary house calls. If anything should occur, for example abroad, a person who needs treatment can contact a physician, emergency ward or hospital, which are associated with the system. The personal code that is stored in the code which, for example, is stuck to the back of a watch, can be read digitally and will thereby give direct information about the patient's medical data and the treatment can be started immediately.

Associated with the centrally placed database, which contains patient data, a complete medicine database can also be arranged, which, for example, can point out harmful interactions between different medicines. Thus, automatic warning can be given if a physician tries to prescribe medicine which react to the other medicines used by the patient. Such an automatic warning can occur when the information is written onto the card or in the database itself.

After treatment, the physician can transfer and load the treatment carried out and medication given into the database, whereupon this is sent by way of a service centre to the patient's regular physician. The database is updated automatically and the patient is sent a new card and new codes if there are any changes with respect to the journal.

This system will give considerable advantages on the human level, make economic sense for industry, insurance companies, etc, and be economic for the society as it cuts down on absenteeism and social security and eases the burden on an already overloaded health system.

The present invention relates to a method for safe transfer of patient data on a data carrier, in that patient data is coded and transferred by means of a network to a central server comprising a database, said patient data being stored in a storage unit in the server, that coded data is transformed and printed on a data carrier which is kept by the patient, and that coded data is read from the data carrier by means of a reading unit and transformed to a legible form by a decoder. The data carrier comprises a sticker adapted to be carried by a user and/or arranged to users personal equipment, and where the sticker comprises a two-dimensional code as information carrier, printed on the sticker.

In a preferred embodiments of the invention, the sticker with the two-dimensional code is adapted for removable adhesion to the back of a watch, a piece of jewellery, etc., and/or to a card which is to be kept together with other cards. The coded data on the data carrier may comprise bar codes, fluorescent labels/tabs, digital chips, etc. Further, the two-dimensional code can be dimensioned to a size of, for example, 10×10mm, on which all available medical information relevant for a person can be stored. For additional security, the code can be adapted to withstand a damage of up to approximately 25%, where the code is automatically self-rehabilitating, whereupon the patient data is still legible after possible defacing or damage.

The decoder, according to the invention, transforms data which is read from the data carrier, and the decoder is connected to a printer for printing of legible patient data.

After a possible medical treatment of a patient, the patient's data is updated and transferred by means of the network to WMC, whereupon data is passed onto the patient's regular physician, the patient data is thereafter updated, coded and transferred by means of the network to the central server, whereupon new data is stored in the storage unit in the server, new coded data is transformed to a two-dimensional form and is printed onto a new data carrier which is kept by the patient.

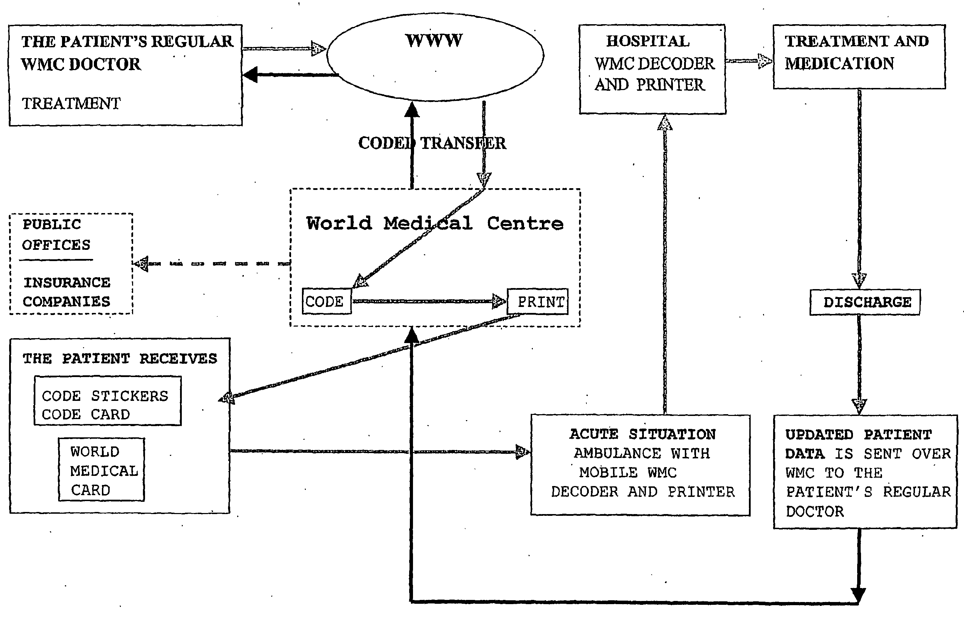

The invention shall now be explained further with the aid of the enclosed figure, which shows the system according the present invention.

The figure shows the construction of the system according to the present invention. Placed centrally is the "World Medical Centre" (WMC), which is comprised of a central database that contains patient data about each patient who is registered in the system. A patient's regular physician will keep the medical journal in his/her office. Essential elements of the information in the journal are transferred in encrypted form, by means of the network to the central database at WMC and are loaded onto a structured medical card. The medical card will contain the patient's diagnosis, illnesses/injuries, use of medication and allergies or reactions to medication. Before transmission, data is automatically encrypted so that the privacy of the patient is ensured.

The registered patient will receive a control-copy of the data, which is loaded into the database for control of the copies that are received by the physician. In addition to the medical card, the patient also receives a data carrier, which, for example, can be a two-dimensional code that contains the same information. The code can be arranged on a self-adhesive means which, for example, can be stuck to the back of a watch, piece of jewellery, etc., and/or on a card to be kept together with other cards. The data carrier can also be comprised of other types of code systems, such as bar codes, fluorescent tabs, digital chips, etc., which will be known to those skilled in the art. The object of the code being in this form is, amongst other things, that it must be able to be fixed to another object for simple storage.

In association with the centrally placed database in WMC, which contains patient data, a complete medicine database can be arranged, which, for example, can point out harmful interactions between different medicines. Thus, automatic warning can be obtained if a physician attempts to prescribe medicines that react to the other medicines the patient is using. Such an automatic warning can occur when the information is written onto the card or in the database itself.

In a present embodiment example, the system is developed with a two-dimensional code as the information carrier in an encrypted form. The code can withstand damage of, for example, about 25% and is automatically self-rehabilitating, so that the information is still legible after possible defacing or damage. One part of the code is used for its own encrypting so that it is not possible to read the information without authorisation. All transfer of the coded information is automatically encrypted or decrypted when transferred internally in the system. This can be, for example, to a printer, a screen or to the central database.

For example, the code can be printed onto a sticker which can be fixed to the back of, for example, a watch, a piece of jewellery, etc. In addition, the code can be printed onto a card and/or on a credit card/cash card. To read the code, a special reading unit has been developed. This reading unit can be in the form of a handheld scanner with a decoder, which can easily be used to read the encrypted information on the sticker.

The code and decoder are constructed such that only those authorised to can read the code. The code does not need to be larger than, for example, 10x10 mm, on which accessible medical information that is essential for a person can be stored.

Thereby, the system comprises a central database, which is placed in a network, preferably an internet-based network. Data, which is stored in the database, is encrypted and printed onto stickers which persons/patients carry with them at all times. In any medical treatment, a scanner combined with a decoder is used, so that others besides the patient's regular physician, i.e. authorised medical personnel, can read the data on the sticker or the card containing the same information.

After treatment, the physician can write the treatment carried out and medication prescribed into his database, whereupon data is thereafter transferred to the central database in WMC and is stored in this. Thereafter, the data can be sent by means of the service centre to the patient's regular physician. The database is updated automatically and the patient will receive a new card and new codes if there are changes with respect to the journal.

## Claims

1. A method of safety transferring patient data on a data carrier, said method comprising the steps of:
(a) coding the patient data and then transferring said coded patient data via a network to a central server comprising a database;
(b) storing said coded patient data in the database of the server;
(c) transforming and printing the coded patient data on a data carrier for retention by the patient; and
(d) using a reading unit to read the coded data from the data carrier and then transforming the coded data to a user legible form by using a decoder,
(e) producing the data carrier as a sticker, said sticker having printed thereon a two-dimensional code for anying the patient medical data, said sticker being suitable for user-keeping by arrangement to a users personal equipment, such as by adhesion to the back of a watch, a piece of jewellery to a card;
(f) after medical treatment of the patient, updating the patient's data;
(g) transferring said updated patent's data via the network to the database In the central server;
(h) passing the updated patent's data further to the patient's regular physician;
(i) coding and then transferring said patient's updated data via the network to the central server for storage thereat; and
(j) transforming said new data to a new two-dimensional coded representation and then printing said representation onto a new data carrier for retention by the patient.

2. A method according to claim 1, wherein the two-dimensional code printed on said sticker comprises at least one of bar codes, fluorescent labels.

3. A method according to claim 2, wherein the two-dimensional code is dimensioned to a size of 10x10 mm.

## Patentansprüche

1. Verfahren zum sicheren Übertragen von Patientendaten auf einem Datenträger, wobei das Verfahren die Schritte umfasst:
(a) Kodieren der Patientendaten und anschließendes Übertragen der kodierten Patientendaten über ein Netzwerk an einen zentralen Server mit einer Datenbank;
(b) Speichern der kodierten Patientendaten in der Datenbank des Servers;
(c) Umsetzen und Drucken der kodierten Patientendaten auf einen von dem Patienten aufzubewahrenden Datenträger; und
(d) Verwenden einer Leseeinheit zum Lesen der kodierten Daten von dem Datenträger und anschließendes Umsetzen der kodierten Daten in eine benutzerlesbare Form mit Hilfe eines Dekoders;
(e) Erzeugen des Datenträgers als ein Sticker, wobei der Sticker einen darauf aufgedruckten zweidimensionalen Kode, welcher die medizinischen Patientendaten beinhaltet, aufweist, wobei der Sticker durch Anbringen an persönliche Sachen eines Benutzers, wie beispielsweise durch Ankleben an die Rückseite einer Uhr, eines Schmuckstücks oder an eine Karte, zur Aufbewahrung durch den Benutzer geeignet ist;
(f) Aktualisieren der Patientendaten nach einer medizinischen Behandlung des Patienten;
(g) Übertragen der aktualisierten Patientendaten über das Netzwerk an die Datenbank in dem zentralen Server;
(h) Weiterleiten der aktualisierten Patientendaten an den Stammarzt des Patienten;
(i) Kodieren und anschließendes Übertragen der aktualisierten Patientendaten über das Netzwerk an den zentralen Server, um dort gespeichert zu werden; und
(j) Umsetzen der neuen Daten in eine neue zweidimensionale kodierte Darstellung und anschließendes Drucken der Darstellung auf einen neuen von dem Patienten aufzubewahrenden Datenträger.

2. Verfahren nach Anspruch 1, wobei der auf den Sticker aufgedruckte zweidimensionale Kode mindestens einen aus Barkodes und fluoreszenten Etiketten ausgewählten Kode umfasst.

3. Verfahren nach Anspruch 2, wobei der zweidimensionale Kode eine Größe von 10x10 mm besitzt.

## Revendications

1. Procédé de transfert de données de patient de manière sûre sur un support d'informations, ledit procédé comprenant les étapes consistant à :
(a) coder les données de patient et ensuite transférer lesdites données de patient codées par le biais d'un réseau vers un serveur central comprenant une base de données ;
(b) mémoriser lesdites données de patient codées dans la base de données du serveur ;
(c) transformer et imprimer les données de patient codées sur un support d'informations pour que le patient les conserve ; et
(d) utiliser une unité de lecture pour lire les données codées à partir du support de données et ensuite transformer les données codées en une forme lisible par un utilisateur en utilisant un décodeur,
(e) produire le support d'informations en tant qu'autocollant, ledit autocollant sur lequel est imprimé un code en deux dimensions pour porter les données médicales du patient, ledit autocollant pouvant être gardé par un utilisateur par un agencement sur un équipement personnel des utilisateurs, tel que par une adhérence au dos d'une montre, d'un bijou, ou sur une carte ;
(f) après un traitement médical du patient, mettre à jour les données du patient ;
(g) transférer lesdites données mises à jour du patient par le biais du réseau vers la base de données dans le serveur central ;
(h) transmettre les données mises à jour du patient au médecin traitant du patient ;
(i) coder et ensuite transférer lesdites données mises à jour du patient par le biais du réseau vers le serveur central pour une mémorisation à ce niveau ; et
(j) transformer lesdites nouvelles données en une nouvelle représentation codée en deux dimensions et ensuite imprimer ladite représentation sur un nouveau support d'informations pour que le patient la conserve.

2. Procédé selon la revendication 1, dans lequel le code en deux dimensions imprimé sur ledit autocollant comprend au moins l'un parmi des codes barres, des étiquettes fluorescentes.

3. Procédé selon la revendication 2, dans lequel le code en deux dimensions est dimensionné selon une taille de 10x10 mm.
